(19) Europäisches Patentamt
European Patent Office
Office européen des brevets

(11) **EP 3 555 632 B1**

(12) **EUROPEAN PATENT SPECIFICATION**

(45) Date of publication and mention
of the grant of the patent:
**10.03.2021 Bulletin 2021/10**

(51) Int Cl.:
***G01N 33/72*** *(2006.01)*     ***G01N 33/86*** *(2006.01)*
***G01N 33/92*** *(2006.01)*     ***C12Q 1/56*** *(2006.01)*

(21) Application number: **17811336.1**

(22) Date of filing: **13.12.2017**

(86) International application number:
**PCT/EP2017/082614**

(87) International publication number:
**WO 2018/109004 (21.06.2018 Gazette 2018/25)**

(54) **DETERMINATION OF BILIRUBIN IN A SAMPLE**

BESTIMMUNG VON BILIRUBIN IN EINER PROBE

DÉTERMINATION DE BILIRUBINE DANS UN ÉCHANTILLON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.12.2016 EP 16204142**

(43) Date of publication of application:
**23.10.2019 Bulletin 2019/43**

(73) Proprietors:
• **Roche Diagnostics GmbH
68305 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **LICHTE, Andrea
6300 Zug (CH)**
• **TOWN, Michael-Harold
82386 Oberhausen (DE)**

(74) Representative: **Altmann Stößel Dick
Patentanwälte PartG mbB
Dudenstrasse 46
68167 Mannheim (DE)**

(56) References cited:
**US-A1- 2009 221 012**

• **M. KOPEC ET AL: "The antithrombin activity of glucuronic esters of bilirubin", JOURNAL OF CLINICAL PATHOLOGY, vol. 14, no. 5, 1 September 1961 (1961-09-01), pages 478-481, XP055437833, GB ISSN: 0021-9746, DOI: 10.1136/jcp.14.5.478**
• **VADIM KOSTOUSOV ET AL: "The Influence of Free Hemoglobin and Bilirubin on Heparin Monitoring by Activated Partial Thromboplastin Time and Anti-Xa Assay", ARCHIVES OF PATHOLOGY & LABORATORY MEDICINE, vol. 138, no. 11, 1 November 2014 (2014-11-01), pages 1503-1506, XP055368969, US ISSN: 0003-9985, DOI: 10.5858/arpa.2013-0572-OA**
• **P MORENO ET AL: "Effects of haemolysis, lipaemia and bilirubinaemia on prothrombin time, activated partial thromboplastin time and thrombin time in plasma samples from healthy dog", RESEARCH IN VETERINARY SCIENCE, vol. 67, 1 January 1999 (1999-01-01), pages 273-276, XP055437842,**
• **JING ZHANG JI ET AL: "Evaluation of the interference of hemoglobin, bilirubin, and lipids on Roche Cobas 6000 assays", CLINICA CHIMICA ACTA, vol. 412, no. 17-18, 1 August 2011 (2011-08-01), pages 1550-1553, XP055368941, AMSTERDAM, NL ISSN: 0009-8981, DOI: 10.1016/j.cca.2011.04.034**
• **DR. P.V. PUPPALWAR ET AL: "Review on "Evolution of Methods of Bilirubin Estimation"", IOSR JOURNAL OF DENTAL AND MEDICAL SCIENCES, vol. 1, no. 3, 1 September 2012 (2012-09-01), pages 17-28, XP055460402, ISSN: 2279-0861, DOI: 10.9790/0853-0131728**

- **SUSAN A. NOWELL ET AL: "Identification of Enzymes Responsible for the Metabolism of Heme in Human Platelets", JOURNAL OF BIOLOGICAL CHEMISTRY, vol. 273, no. 50, 11 December 1998 (1998-12-11), pages 33342-33346, XP055460405, US ISSN: 0021-9258, DOI: 10.1074/jbc.273.50.33342**

**Description**

The present disclosure relates to a method for determining a non-anticoagulant interferent in a blood-derived sample of a subject, said method comprising a) determining a value of a coagulation time-related parameter in said sample; b) comparing the value of the coagulation time-related parameter determined in a) to a value of said coagulation time-related parameter determined in at least one reference sample; and c) based on the result of comparison step b), determining said non-anticoagulant interferent in a blood-derived sample of a subject. Further, the present disclosure relates to a method for identifying a blood-derived sample having insufficient quality, said method comprising determining non-anticoagulant interferent(s) according to the aforesaid method, and to devices, kits, and uses related thereto.

Analysis of blood samples in clinical chemistry is often confounded by the presence of compounds in the samples which interfere with the assays used. Most prominent confounders are hemoglobin, mostly released by hemolysis in the sample; bilirubin, in particular in samples of patients suffering from icterus, and increased lipid concentration, in particular in samples from lipidemic patients; this is also known as hemolysis, icterus and lipidemia (HIL) interference. As an example, antithrombin activity of glucuronic esters of bilirubin was reported already in 1961 (Kopec et al. (1961), J Clin Path 14:478.

Detecting and reporting HIL interference has traditionally been done by visual inspection of individual specimens by laboratory personnel; however, this process is highly subjective and variable. Recently, clinical chemistry analyzers were launched that offer automated detection of the HIL status on serum or plasma samples and have reported HIL turbidity index values (cf. e.g. Shin et al. (2014), Ann Lab Med 34:307; Boyd et al. (2015), Clin Chim Acta 450:3). HIL indices (also known as the threshold levels) are semi-quantitative estimates of HIL interference, using spectrophotometric measurement and mathematical correction to determine the level of interference. In clinical chemistry analyzers those indices are usually defined as the lowest concentration of HIL that interferes with the analyses, yielding a bias >10%.

HIL indices can be used in pre-analytical algorithms to reject samples for analysis if they are above the specified alert indices that are recommended by the manufacturers of their respective in vitro diagnostic kits or analyzers. The pre-defined HIL indices are analyzer specific and basically based on absorbance readings at several selected wavelengths. Since the absorbance spectra of bilirubin, hemoglobin, and lipemia overlap to some extent, the calculation of HIL indices can only be semi-quantitative. For instance the absorbance spectrum of hemoglobin overlaps most of the spectrum of bilirubin, therefore estimating the degree of icterus in the presence of hemolysis or vice versa is a difficult challenge for HIL measurement when photometric methods are used.

Bilirubin quantification is mainly performed based on a diazo-coupling reaction (US 6,326,208 B1). Bilirubin and diazobenzene-p-sulphonic acid give a colored product (azobilirubin) which is red in neutral and blue in alkaline solution (van den Bergh reaction). Unconjugated bilirubin (biliu) reacts slowly with the azo dye because it is insoluble in water at physiologic pH and temperature. Bilirubin conjugated to glucuronic acid (bilic) is watersoluble and reacts quickly. Biliu reaction can be accelerated by so-called accelerator substances, e.g. methanol or caffeine (Walters and Gerarde (1970), Microchemical Journal 15:231; Valdes et al. (1979), Annal. Clin. Lab. Science 9(3):251). The reaction is usually stopped by addition of ascorbic acid and the extinction of the dye is read in a spectrophotometer at 540 nm. Typically, these assays require the use of hazardous chemical compounds. Efforts trying to replace such compounds were made, e.g. by using a bilirubin-inducible fluorescent protein from Japanese eel muscle (Iwatani et al. (2016), Scientific Reports, doi 10.1038/srep28489). An enzymatic method which employs bilirubin oxidase is also known. Bilirubin oxidase decreases the absorbance at 450 nm when it catalyzes oxidation of bilirubin to biliverdin. A major disadvantage of the conventional methods (diazo method, bilirubin oxidase method, or spectrophotometry) is that the results are affected by turbidity in serum samples. For example, the bilirubin oxidase method measures bilirubin levels by monitoring a change in absorbance at 450 nm, which is affected by turbidity due to hemoglobin or lipid emulsion.

Given the disadvantages of the methods known in the art such as the use of toxic chemical compounds and problems caused by turbid samples, there is a need for improved methods for determining the presence of interfering compounds in blood samples, in particular compounds interfering with coagulation and/or clinical chemistry assays, avoiding the drawbacks of the prior art. This problem is solved by the means and methods disclosed herein.

Accordingly, the present invention relates to a method for determining conjugated bilirubin in a blood-derived sample of a subject according to claim 1; further disclosed is a method for determining a non-anticoagulant interferent in a blood-derived sample of a subject, said method comprising

a) determining a value of a coagulation time-related parameter in said sample;
b) comparing the value of the coagulation time-related parameter determined in a) to a value of said coagulation time-related parameter determined in at least one reference sample; and
c) based on the result of comparison step b), determining said non-anticoagulant interferent in a blood-derived sample of a subject.

The method for determining conjugated bilirubin of the present invention is an in vitro method. Moreover, it may comprise steps in addition to those explicitly mentioned above. For example, further steps may relate, e.g., to providing a sample for step a), or providing an appropriate reference sample for step b). Further steps the method for determining may comprise are specified herein below. Moreover, one or more of said steps may be performed by automated equipment.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value ± 20%, more preferably ± 10%, most preferably ± 5%.

The term "determining a non-anticoagulant interferent", as used herein, relates to providing an indication on the presence and/or the amount of at least one non-anticoagulant interferent in a sample. In an embodiment, determination is quantitative determination, in a further embodiment is absolute quantification of the concentration of a non-anticoagulant interferent in a sample, which can be provided e.g. as a value of weight per volume, activity per volume, or the like; and/or determination is relative quantification of the concentration of a non-anticoagulant interferent, e.g. compared to a reference value, which can be provided e.g. in arbitrary units per volume or as a fraction of the reference value, e.g. in percent. In a further embodiment, determining a non-anticoagulant interferent is qualitative determination, i.e., in an embodiment, is evaluating whether a non-anticoagulant interferent is present in a sample in an amount or concentration above the detection limit. In a further embodiment, determining a non-anticoagulant interferent is semi-quantitative determination, i.e. is evaluating whether a non-anticoagulant interferent is present in a sample in an amount or concentration above a predetermined threshold, e.g. a quality threshold, i.e. a threshold established to decide whether a sample is of sufficient quality for further analysis, in an embodiment as specified herein below. The value of the predetermined threshold will depend on the specific application of the method envisaged. The skilled person knows how to establish one or more threshold values, e.g. by measuring the values of the corresponding parameter in one or more reference sample(s) as specified herein below. In an embodiment, determining a non-anticoagulant interferent is evaluating whether at least one non-anticoagulant interferent is present in a sample at a concentration exceeding at least one threshold. As will be understood by the skilled person, evaluating whether at least one non-anticoagulant interferent is present in a sample at a concentration exceeding at least one threshold may comprise determining, for each non-anticoagulant interferent of interest, whether its concentration in a sample exceeds a predetermined threshold; in an embodiment, at least one predetermined threshold is used per non-anticoagulant interferent of interest in such case. In a further embodiment, evaluating whether at least one non-anticoagulant interferent is present in a sample at a concentration exceeding at least one threshold comprises, in an embodiment is, determining whether the non-anticoagulant interferent(s) present in said sample modulate a coagulation time-related parameter to such an extent that the sample should be excluded from determining said coagulation time-related parameter and/or from clinical chemistry. Thus, in an embodiment, determining a non-anticoagulant interferent does not comprise identifying a non-anticoagulant interferent in a sample; such determining is in particular envisaged if determining a non-anticoagulant interferent in a sample is used in quality control, e.g. in pre-analysis quality control of blood-derived samples as specified herein below. In an embodiment, quantitative and semi-quantitative determination comprise comparing the value determined in a sample to more than one reference values. In an embodiment, determining a non-anticoagulant interferent comprises comparing the value of the coagulation time-related parameter determined in a sample to at least two reference values, e.g. a negative control and a positive control. In a further embodiment, determining a non-anticoagulant interferent comprises establishing a calibration curve from at least three, in an embodiment at least four, in a further embodiment at least five reference samples having predetermined, non-identical concentrations of non-anticoagulant interferent. In an embodiment, the concentration of non-anticoagulant interferent of at least one reference sample is below the detection limit.

As used herein, the term "reference value" relates to the value of a parameter determined in a reference sample. In an embodiment, said parameter is a coagulation time-related parameter. In an embodiment, a reference value is available from earlier determinations and, in an embodiment, is provided e.g. in a database; in a further embodiment, a reference value is determined concomitantly to determining the value of at least one parameter in a sample of interest. The term "corresponding parameter" relates to a parameter providing essentially the same information as the parameter it corresponds to, potentially including standard mathematical operations. E.g. the value of an amount determined in a known volume of a sample corresponds to a value of a concentration. In an embodiment, a corresponding parameter is the same parameter.

A "reference sample", as the term is used herein, is a sample comprising the compounds required for a coagulation reaction to occur, in an embodiment in the concentrations these compounds are found in a blood-derived sample, and comprising a known amount of non-anticoagulant interferent(s). In an embodiment, the term "known amount of non-anticoagulant interferent(s)" relates to an amount of non-anticoagulant interferent(s) known semiquantitatively. Said semiquantitative amount may, in an embodiment e.g. be an amount above or below the detection limit, or an amount above or below a quality threshold. In a further embodiment, the amount of non-anticoagulant interferent(s) is known quantitatively, i.e. has been determined as a relative or absolute amount or concentration. The reference sample may be an artificial sample; in an embodiment, the reference sample is or is based on a natural sample, in an embodiment is plasma, in a further embodiment is citrate plasma, as specified below. In an embodiment, a reference sample is a sample allowing, in an embodiment semi-quantitatively, determining whether a sample comprises non-anticoagulant interferent(s) or not. Thus, as used herein, in a reference sample, the absolute concentration of non-anticoagulant interferent(s) need not necessarily be known. In an embodiment, a reference sample is a sample allowing quantitative determination of non-anticoagulant interferent(s) in a sample. In such case, in an embodiment, the absolute concentration of non-anticoagulant interferent(s) in the reference sample is known.

In an embodiment, the reference sample is known not to comprise a non-anticoagulant interferent in detectable amounts; such a reference sample can e.g. be provided by providing an artificial reference sample without non-anticoagulant interferent being added; or with non-anticoagulant interferent being added at a concentration below the detection limit; or such a reference sample can be provided by determining the amount of non-anticoagulant interferent(s) in a blood derived sample by a method different from the method of the present invention and by selecting a sample for which absence of non-anticoagulant interferent(s) is detected; or by adjusting the concentration of the non-anticoagulant interferent to be below the detection limit. In an embodiment, such a sample may also be provided by the method of the present invention using a reference sample identified to not comprise a non-anticoagulant interferent previously and by selecting a sample for which absence of non-anticoagulant interferent(s) is detected. Furthermore, a reference sample may be a sample from an apparently healthy subject or from a population of apparently healthy subjects, wherein the term "apparently healthy subject", as used herein, relates to a subject not having been diagnosed to suffer from a coagulation defect. In a further embodiment, the reference sample is a mixture of samples from a population of subjects, provided that the incidence of samples comprising a non-anticoagulant interferent in said population of subjects is low enough to statistically ensure that the concentration of non-anticoagulant interferent(s) does not significantly deviate from the concentration of a non-anticoagulant interferent(s) in a sample of a healthy subject. A reference sample known not to comprise a non-anticoagulant interferent in detectable amounts may e.g. be used as a negative control sample.

Alternatively or additionally, a "reference sample" is a (further) sample known to comprise a non-anticoagulant interferent in a detectable amount. Such a reference sample can e.g. be provided by providing an artificial reference sample and by adding a known amount of non-anticoagulant interferent to a said artificial sample. Also, such a sample may be provided by adding a known amount of non-anticoagulant interferent to a blood-derived sample, in an embodiment to a sample known not to comprise a non-anticoagulant interferent as specified above. In a further embodiment, such a reference sample is a blood-derived sample obtained from a subject known to comprise non-anticoagulant interferent(s); in an embodiment, the presence and/or concentration of said non-anticoagulant interferent(s) is established by a method different from the method of the present invention. In an embodiment, such a reference sample may also be provided by the method of the present invention using a reference sample identified to comprise a non-anticoagulant interferent previously and by selecting a sample identified to comprise non-anticoagulant interferent(s). A reference sample known to comprise a non-anticoagulant interferent in a pre-determined, detectable amount may e.g. be used as a positive control sample; or a reference sample known to comprise a non-anticoagulant interferent in a pre-determined, detectable amount may be used as a quality control sample, e.g. to determine precision of measurements.

The term "calibrator sample", as used herein, relates to a reference sample wherein the amount or concentration of non-anticoagulant interferent(s) is known. In an embodiment, a calibrator sample is a blood-derived sample comprising a predetermined amount of non-anticoagulant interferent(s). In an embodiment, at least two, in a further embodiment at least three, in a further embodiment at least four calibrator samples are provided, said calibrator samples comprising pre-defined, non-identical concentrations of non-anticoagulant interferent(s). In an embodiment, said calibrator samples are provided by adjusting the concentration of said non-anticoagulant interferent(s) in said calibrator samples by admixing non-anticoagulant interferent(s) into said calibrator sample. In a further embodiment, at least two, in a further embodiment at least three, in a further embodiment at least four calibrator samples are derived from one calibrator stock solution. In a further embodiment, at least two, in a further embodiment at least three, in a further embodiment at least four calibrator samples are derived from two calibrator stock solutions, e.g. from one calibrator stock solution known not to comprise anticoagulant interferent(s), and one calibrator stock solution comprising anticoagulant interferent(s) at the highest concentration used for calibration.As will be understood by the skilled person, in an embodiment, at least one calibrator sample comprises non-anticoagulant interferent(s) at a concentration below the detection limit, and at least one calibrator sample comprises non-anticoagulant interferent(s) at a pre-determined concentration above the detection limit.

The term "interferent", as used herein, relates to an agent modulating a coagulation time-related parameter in a blood derived sample of a subject. As is known to the skilled person, coagulation time can be modified by the presence of an anticoagulant in a sample. An "anticoagulant", as the term is used herein, is a chemical compound used in therapeutic applications to modulate, in an embodiment to prolong, coagulation in a subject. Anticoagulants are known to the skilled person and include heparin, e.g. unfractionated heparin or low molecular weight heparin; factor Xa inhibitor, e.g. synthetic pentasaccharide or direct factor Xa inhibitor; direct thrombin inhibitor; or vitamin K antagonists. Further anticoagulants are 4-hydroxycoumarin, e.g. acenocoumarol, dicumarol, ethyl biscoumacetate, phenprocoumon, warfarin, coumatetralyl, difenacoum, flocoumafen, bromadiolone, tioclomarol, brodifacoum, and/or phenprocoumon (marcumar, (RS)-4-Hydroxy-3-(1-phenylpropyl)cumarin); e.g. warfarin ((RS)-4-Hydroxy-3-(3-oxo-I-phenylbutyl)-2H-chromen-2-one); e.g. (-)-warfarin ((S)-4-Hydroxy-3-(3-oxo-1-phenylbutyl)-2H-chromen-2-one), e.g. phenprocoumon (marcumar, (RS)-4-Hydroxy-3-(1-phenylpropyl)cumarin). Further anticoagulants are Phenindione, Clorindione, and/or Diphenadione.

Further interferents are "non-anticoagulant interferents", i.e. compounds which are not anticoagulants as specified above, however, have the activity of modulating a coagulation time-related parameter in a sample. In a disclosure, said non-anticoagulant interferent is a compound produced by body cells of a subject. The non-anticoagulant interferent is conjugated bilirubin.

The term "coagulation time-related parameter" relates to a parameter indicating the time required for a blood-derived sample to clot. Accordingly, the term "coagulation time-related parameter" relates to a parameter indicating or correlating with the time required for a blood-derived sample to clot or to a parameter derived therefrom. In an embodiment, a coagulation-time related parameter is derived from the aforesaid parameter by standard operations of mathematics, physics and/or chemistry. Usually, in coagulation laboratories, e.g. activated Prothrombin Time (APTT) reagents and Thrombin Time (TT) reagents are used to screen for coagulation abnormalities and to monitor the treatment effects of anticoagulants in therapy. Prolonged APTT may indicate use of heparin (or contamination of the sample), antiphospholipid antibody (especially lupus anticoagulant), coagulation factor deficiency (e.g. hemophilia), Sepsis including coagulation factor consumption, or presence of antibodies against coagulation factors (factor inhibitors). According to the invention, the coagulation time-related parameter is a thrombin time. Methods for determining coagulation time are well-known in the art; e.g., the coagulation time-related parameter is determined in a chromogenic assay, with a mechanical assay, by determining coagulation clot mass over time, by determining a viscosity-related parameter over time, e.g. oscillation of a steel bead embedded in a coagulation reaction (Hubbuch et al. (1996), Clin. Lab. 42: 637-640), or with a photo-optical assay, all of which are known to the skilled person. According to the invention, the coagulation time-related parameter is determined with a photo-optical assay, in a further embodiment by photometry, in a further embodiment by nephelometry or by turbidimetry, in a further embodiment by turbidimetry. Thus, in an embodiment, the coagulation time-related parameter is determined by determining at least one transmission-related parameter over time as specified elsewhere herein. As is understood by the skilled person, the transmission-related parameter is, in an embodiment, determined at least twice at two non-identical time points after eliciting coagulation.

The term "blood-derived sample", as used herein, relates to blood, or to a sample material derived from blood, comprising at least the clotting factors comprised in blood required for coagulation. In an embodiment, the blood-derived sample is plasma, in a further embodiment is citrate plasma. Methods for obtaining blood derived samples from a subject are known to the skilled person and include, in an embodiment, arterial or venous puncture, and puncture of the skin.

The term "subject", as used herein, relates to a vertebrate animal, in an embodiment a mammal, in a further embodiment a human. In an embodiment, the subject is an apparently healthy subject. In a further embodiment, the subject is a subject not being administered anticoagulation therapy, i.e. is a subject not being treated with an anticoagulant as specified herein above. In a further embodiment, the subject is known not to suffer from a coagulopathy. In a further embodiment, the subject is a subject with an increased risk for developing bilirubin encephalopathy, in particular the subject is a newborn, in an embodiment a human newborn. In a further embodiment, the subject is a human newborn with an age of less than two months, in an embodiment of less than one month, in an embodiment of less than two weeks. In a further embodiment, the subject is a preterm infant, in an embodiment a human infant with delivery at less than 37 weeks' gestation.

In an embodiment, the method comprises additional steps and/or substeps. As is known to the skilled person, coagulation requires the presence of calcium ions; on the other hand, clinical samples for coagulation testing usually contain calcium ion chelators to prevent premature coagulation. Thus, in an embodiment, step a), i.e. determining a value of a of thrombin time in the sample, comprises a substep a0) contacting said sample with an agent providing calcium ions, in an embodiment shortly before or simultaneously to contacting said sample with an agent eliciting coagulation. In an embodiment, step a), i.e. determining a value of a of thrombin time in the sample, comprises a substep a1) contacting said sample with an agent eliciting coagulation being thrombin. In a further embodiment, step a) comprises a substep a2) determining a first value of a transmission-related parameter at a first time point after eliciting coagulation in said sample, and a substep a3) determining a second value of a transmission-related parameter at a second time point after eliciting coagulation in said sample. As noted herein above, the first time point and the second time point are non-identical. In a further embodiment, step a) comprises further substeps of determining further values of a transmission-related parameter at further time points after eliciting coagulation in said sample. Thus, in an embodiment, step a) comprises a multitude of substeps of determining a value of a transmission-related parameter at a multitude of non-identical time points after eliciting coagulation in said sample, i.e. comprises, in an embodiment, determining values of a transmission-related parameter after a multitude of intervals, in an embodiment at regular intervals. Thus, in an embodiment, values of a transmission-related parameter are determined at least every 10 s, in a further embodiment at least every 8 s, in a further embodiment at least every 5 s, in a further embodiment at least every 3 s, in a further embodiment at least every 2 s, in a further embodiment at least every second, in a further embodiment at least every 0.2 s. In a further embodiment, step a) comprises continuously determining values of a transmission-related parameter. As is understood by the skilled person, determining a transmission-related parameter, in an embodiment, comprises at least establishing a signal correlating with the transmission-related parameter and actual recording values of said signal. While a signal correlating with the transmission-related parameter may be provided truly continuously e.g. as a voltage measured in a photocell of a photometer, or may be provided in discrete measurements, e.g. by moving a well of a multi-well plate into the light path of a photometer, recording of values of said signal will be in discrete steps at given points in time. Nonetheless, as used herein, the term "continuously determining values of a transmission-related parameter" relates to determining values of a transmission-related parameter at least every 0.1 s. Thus, the term relates to recording values of the transmission-related parameter at the aforesaid frequency. In a further embodiment, step a) comprises substep a4) determining a value of a thrombin time in said sample based on the first value of a transmission-related parameter determined in substep a2) and on the second value of a transmission-related parameter determined in substep a3). As will be understood by the skilled person, in case more than two values of a transmission-related parameter are determined, determining a value of a thrombin time is based on at least two of said values of transmission-related parameter determined; in an embodiment, determining a value of a thrombin time is based on more than two of said values of transmission-related parameters determined, in an embodiment based on three or more, in a further embodiment based on four or more, in a further embodiment based on five or more, in a further embodiment based on all values of said transmission-related parameter determined. Thus, in an embodiment, determining a value of a thrombin time parameter is based on the values of a thrombin time determined in said sample. Accordingly, in an embodiment, step a) comprises the substeps a1) contacting said sample with thrombin; a2) determining a first value of a transmission-related parameter in said sample at a first time point after eliciting coagulation in said sample; a3) determining a second value of a transmission-related parameter in said sample at a second time point after eliciting coagulation in said sample; and a4) determining a thrombin time based on the results of substeps a2) and a3). In a further embodiment step a) comprises the substeps a1) contacting said sample with thrombin; a2) determining a multitude of values of a transmission-related parameter in said sample at a multitude of non-identical time points after eliciting coagulation in said sample; and a3) determining a thrombin time based on the results of substep a2).

The term "transmission-related parameter" relates to a parameter indicating or correlating with the ratio of transmitted light versus incident light of a sample or to a parameter derived therefrom. In an embodiment, a transmission-related parameter is derived from the aforesaid ratio by standard operations of mathematics, physics and/or chemistry. Accordingly, in an embodiment, the transmission-related parameter is a transmission coefficient, an extinction coefficient, a transmittance, an absorbance, a light scattering, or an absorption. Moreover, in an embodiment, the transmission-related parameter is a value derived from one of the aforesaid parameters by a standard mathematical operation, e.g. to correct for a dilution applied to a sample, for a calibration factor, and the like. Means and methods for determining a transmission-related parameter in a blood sample are known to the skilled person and are, in an embodiment, transmission measurement, nephelometry, or turbidimetry. The sample may, for determining a transmission-related parameter, be comprised in a well of a multi-well plate. In a further embodiment, the sample is comprised in a cuvette; cuvettes can be used as single cuvettes or in arrangements for multiple cuvettes such as cuvette rotors or rack-based systems packed with cuvettes. In an embodiment, transmission-related parameters are determined by turbidimetry. It is understood by the skilled person that a transmission-related parameter may be determined at a specific wavelength; thus, in an embodiment, said transmission-related parameter is determined at a wavelength of visible light, in an embodiment at a wavelength of from 300 nm to 700 nm, in an embodiment of from 300 nm to 600 nm or of from 500 nm to 700 nm. In an embodiment, the transmission-related parameter is determined at a wavelength of from 550 nm to 650 nm, in a further embodiment of from 600 nm to 650 nm, in a further embodiment at a wavelength of 625 ± 10 nm, in a further embodiment at a wavelength of 625 nm. It is also known to the skilled person that the aforesaid transmission-related parameters may be used to determine a coagulation time-related parameter, e.g. by identifying a plateau in the graphical representation of values over time and thereof deducing a predefined percentage of signal height and/or by extrapolating from the slope of a graph representing the change of the values of said transmission-related parameter over time.

In an embodiment, step b), i.e. comparing the value of the thrombin time determined in step a) as specified herein above to a value of said thrombin time determined in a reference sample, comprises a substep b1) providing at least two, in an embodiment at least three, in a further embodiment at least five, calibrator samples having predetermined, non-identical concentrations of said conjugated bilirubin. In an embodiment, step b) comprises a substep b2) determining a value of said thrombin time for each of said calibrator samples, in an embodiment according to the substeps of step a). In a further embodiment, step b) comprises a substep b3) providing a calibration curve based on the values determined in substep b2). Thus, in an embodiment, step b) comprises substeps b1) providing at least two, in an embodiment at least three, in a further embodiment at least five, calibrator samples having predetermined, non-identical concentrations of said conjugated bilirubin; b2) determining a value of said thrombin time for each of said calibrator samples, in an embodiment according to the substeps of step a); and b3) providing a calibration curve based on the values determined in substep b2).

In an embodiment, the blood-derived sample is a sample of a subject suspected not to comprise interferents, in particular anticoagulants, e.g. a sample from an apparently healthy subject as specified herein above. In a further embodiment, the blood-derived sample is a sample from a subject known or suspected to be treated with an anticoagulant. In such case, in an embodiment, the method for determining conjugated bilirubin comprises contacting said sample with a compound selected from hexadimethrine bromide, protamine and heparinase. Alternatively or additionally, in an embodiment, the method comprises contacting said sample with an anti-fibrinolytic agent, in an embodiment tranexamic acid or an anti-fibrinolytic derivative thereof or aprotinin or an anti-fibrinolytic derivative thereof.

The method is a method comprising determining conjugated bilirubin. Thus, in an embodiment, the method comprises contacting an aliquot of said sample with bilirubin oxidase (EC 1.3.3.5) and determining a value of said coagulation time-related parameter in said bilirubin oxidase treated aliquot, in an embodiment according to the method according to claim 1. In a further embodiment, the method further comprises determining total bilirubin. Thus, in a further embodiment, the method is a method comprising determining total bilirubin. Methods for determining total bilirubin are known in the art, e.g. from the documents cited herein above. In a further embodiment, the method for determining a non-anticoagulant interferent is a method comprising determining total bilirubin and calculating free bilirubin from the values determined for total bilirubin and conjugated bilirubin.

In an embodiment, the method for determining a conjugated bilirubin further comprises contacting an aliquot of said sample with a beta-glucuronidase (EC 3.2.1.31) and determining a value of the thrombin time, in said bilirubin oxidase treated aliquot, in an embodiment according to the method of claim 1. In an embodiment, the beta-glucuronidase is a beta-glucuronidase using conjugated bilirubin as a substrate; suitable beta-glucuronidases are known in the art and include those from bacteria, in particular intestine bacteria including Escherichia coli; mammalian hepatic beta-glucuronidases, e.g. human or bovine hepatic beta-glucuronidases; Helix pomatia beta-glucuronidase; and Abalone beta-glucuronidase.

In an embodiment, the method for determining a conjugated bilirubin comprises further steps aiding in establishing the identity of the interferent. In an embodiment, the method further comprises determining turbidity and/or absorption of the sample before eliciting coagulation. As will be understood by the skilled person, an increased turbidity, compared to a reference not comprising increased amounts of lipids, before eliciting coagulation, in an embodiment, is indicative that the non-anticoagulant interferent is a lipid as specified herein above; and an increased absorption, in an embodiment measured at a wavelength of from 400 nm to 600 nm, compared to a reference not comprising increased amounts of hemoglobin, is indicative that the non-anticoagulant interferent is hemoglobin. As indicated above, a difference in the coagulation time-related parameter between an aliquot of a sample and a further aliquot of said sample having been treated with bilirubin oxidase is indicative of the non-anticoagulant interferent being bilirubin. In an embodiment, the method for determining a non-anticoagulant interferent comprises further and/or additional steps aiding in establishing the identity of the interferent, said steps being known to the skilled person, e.g. enzymatic methods, chemical detection reactions, staining methods, e.g. with lipophilic agents, and the like.

Advantageously, it was found in the work underlying the present invention, that compounds naturally occurring in blood-derived samples, in particular bilirubin, hemoglobin, and lipids, can interfere with coagulation and that by means of this interference interfering agents can be quantitated via determining a coagulation time-related parameter. Furthermore, since the interfering agents are frequently observed confounders of coagulation tests and of clinical chemistry assays, blood-derived samples comprising elevated amounts of these compounds are preferably excluded from such analysis to avoid generation of false results.

The definitions made above apply mutatis mutandis to the following. Additional definitions and explanations made further below also apply for all embodiments described in this specification mutatis mutandis.

The present invention further relates to a method for identifying a blood-derived sample having insufficient quality according to claim 9. Further disclosed is a method for identifying a blood-derived sample having insufficient quality, said method comprising

    A) determining non-anticoagulant interferent(s) according to the present invention;
    B) comparing the result of step A) to a quality reference; and
    C) based on the result of comparison step B), identifying a sample having insufficient quality.

As used herein, the term "blood-derived sample having insufficient quality" relates to a blood-derived sample comprising non-anticoagulant interferent(s) in amounts interfering with at least one assay of clinical chemistry and/or coagulation measurements. In an embodiment, the term relates to a sample comprising conjugated bilirubin in amounts interfering with coagulation measurements. In an embodiment, the term relates to samples in for which, if analyzed according to standard protocols, at least one clinical chemistry value and/or coagulation measurement value significantly deviating from the actual value would be measured or would be expected to be measured. Thus, in an embodiment, a blood-derived sample having insufficient quality is a sample which should not be analyzed according to standard protocols to avoid false results, or should be pre-treated before such analysis to reduce the amount or to remove conjugated bilirubin. E.g., in an embodiment, in case the sample is identified to comprise a concentration of bilirubin above a threshold, said sample may be treated with bilirubin oxidase before further analysis. Also, in disclosure, in case the sample is identified to comprise a concentration of lipids above a threshold, said sample may be treated with an appropriate lipidase before further analysis, may be subjected to centrifugation to remove lipid droplets, or the like. In a further embodiment, a sample identified as having insufficient quality is excluded from clinical chemistry analysis and/or coagulation measurements.

As specified herein above, a threshold may be a defined threshold value or a threshold range. However, it is also envisaged that, in an embodiment, the predetermined threshold is provided by providing a reference sample comprising a threshold amount of conjugated bilirubin; e.g. a reference sample comprising a highest tolerable concentration of conjugated bilirubin may be provided. In principle, the actual concentration of conjugated bilirubin in said reference sample does not have to be known, since a sample comprising a threshold concentration may also be identified empirically. In a further embodiment, the threshold is a threshold value, in an embodiment a threshold concentration.

According to the invention, the result of determining conjugated bilirubin may be compared to a quality reference. As used herein, the term "quality reference" relates to a reference sample or reference value enabling a decision whether a given blood-derived sample is of sufficient quality or not. In an embodiment, a quality reference is a reference sample comprising a highest tolerable concentration of conjugated bilirubin and values of corresponding parameters are compared between the blood-derived sample and the quality reference sample. In a further embodiment, the quality reference is a reference value, which may be pre-determined or may be determined from e.g. a calibration curve. Thus, in an embodiment, the quality reference is a quality reference range or a quality reference curve. In a further embodiment, the lower limit of said quality reference is the value of the coagulation time-related parameter in a normal sample -20%, in an embodiment -10%, in a further embodiment -7.5%. In a further embodiment, the upper limit of said quality reference is the value of the coagulation time-related parameter in a normal sample +20%, in an embodiment +10%, in a further embodiment +7.5%. In a further embodiment, the quality reference is a quality reference range being the value of the coagulation time-related parameter in a normal sample ±20%, in an embodiment ±10%, in a further embodiment ±7.5%. In a further embodiment, the lower limit of the quality reference is the lower limit of normal of the value of the coagulation time-related parameter in a normal sample. In a further embodiment, the upper limit of said quality reference is the upper limit of normal of the value of the coagulation time-related parameter in a normal sample.

In disclosure, the non-anticoagulant interferent is hemoglobin; in such case, a blood-derived sample having insufficient quality is identified, e.g., if the hemoglobin concentration determined is higher than 20 mg/dL, or higher than 50 mg/dL.

In a further embodiment, the non-anticoagulant interferent is conjugated bilirubin; in such case, a blood-derived sample having insufficient quality is identified, in an embodiment, if the conjugated bilirubin determined is higher than 1 mg/dl, in an embodiment higher than 2.5 mg/dL.

In a further disclosure, the non-anticoagulant interferent is lipid, e.g. a mixture comprising triglycerides; in such case, a blood-derived sample having insufficient quality is identified, e.g. if the lipid concentration determined is higher than 100 mg/dL, or is higher than 250 mg/dL. As is understood by the skilled person, increased lipid concentrations in a blood-derived sample may lead to turbidity; accordingly, a blood-derived sample having insufficient quality is identified, e.g., in case the value of a transmission-related parameter in said sample before coagulation is higher than a transmission reference. The term "transmission reference", as used herein, relates to a value determined in a reference sample as specified herein above known to comprise a maximally acceptable concentration of lipids or to a reference value corresponding to said maximally acceptable concentration of lipids. In a disclosure, the transmission reference value is the value of the transmission-related parameter in a normal sample +20%, in an embodiment +10%, in a further disclosure +7.5%. In a further disclosure, the transmission reference value is the upper limit of normal of the value of the transmission-related parameter in a normal sample.

Accordingly, in an embodiment, the method for identifying a blood-derived sample having insufficient quality comprises evaluating at least one of the following conditions and the sample is identified as having insufficient quality if at least one of the following conditions is met:

  i) the value of the coagulation time-related parameter in said sample is lower than a quality reference;
  ii) the value of the coagulation time-related parameter in said sample is higher than a quality reference and the value of the coagulation time-related parameter is significantly lower after treatment of said sample with bilirubin oxidase;
  iii) the concentration of conjugated bilirubin determined is higher than 1 mg/dl, in an embodiment higher than 2.5 mg/dL.

The present invention also relates to a device for determining conjugated bilirubin according to claim 11; further disclosed is a device for determining a non-anticoagulant interferent in a blood-derived sample, comprising an analysis unit and an evaluation unit, wherein

  (I) said analysis unit is adapted for determining a value of a coagulation time-related parameter in said sample, and
  (II) said evaluation unit comprises a memory unit comprising tangibly embedded an algorithm for comparing the coagulation time-related parameter determined by the analysis unit to a quantification reference for said non-anticoagulant interferent.

The term "device", as used herein, relates to a system of means comprising at least the means described operatively linked to each other as to allow the determination. Typical means for determining a coagulation time-related parameter are known in the art and have been described herein above in the context of the methods of the invention. The means for determining a value of thrombin time are means for determining a transmission-related parameter, which are known in the art and are for example described in Hubbuch et al. (1996), Clin. Lab. 42: 637-640. How to link the means of the device in an operating manner will depend on the type of means included into the device. In an embodiment, the means are comprised by a single device. However, it is also contemplated that the means of the current invention, in an embodiment, may appear as separate devices and are, in a further embodiment, packaged together as a kit. The person skilled in the art will realize how to link the means without further ado. Preferred devices are those which can be applied without the particular knowledge of a specialized technician. In an embodiment, the device is adapted to include an additional feature as described herein.

In an embodiment, the device includes an analysis unit adapted for determining a value of thrombin time and an evaluation unit for processing data received from the analysis unit and/or from the database for providing determination of a conjugated bilirubin. Determining a conjugated bilirubin comprises determining a thrombin time based on a transmission-related parameter determined by the analysis unit as specified elsewhere herein. Accordingly, in an embodiment, the analysis unit is adapted to measure a multitude of values of said transmission-related parameter of the same sample at time intervals, in an embodiment at regular time intervals, in a further embodiment as specified elsewhere herein. In an embodiment, the analysis unit is further adapted for determining values of thrombin time in reference samples, in an embodiment in calibration samples. In a further embodiment, the analysis unit is further adapted for automatically providing calibration samples, in an embodiment for automatically providing at least two, in an embodiment at least three, in a further embodiment at least five calibration samples, in a further embodiment from one or from two calibration stock solution(s). In a further embodiment, the analysis unit comprises an optical unit adapted for determining a transmission-related parameter at a wavelength of from 350 nm to 700 nm, in an embodiment of from 600 nm to 650 nm, in a further embodiment at a wavelength of 625 ± 10 nm, in a further embodiment at a wavelength of 625 nm, in a further embodiment of from 400 nm to 450 nm. In a further embodiment, the analysis unit comprises an optical unit adapted for determining a transmission-related parameter exclusively at a wavelength of from 350 nm to 700 nm, in an embodiment of from 600 nm to 650 nm, in a further embodiment at a wavelength of 625 ± 10 nm, in a further embodiment at a wavelength of 625 nm, in a further embodiment of from 400 nm to 450 nm.

The evaluation unit of the device comprises a memory unit comprising tangibly embedded an algorithm for performing steps b) and c) of the method of claim 1. In a further embodiment, the memory unit comprises a calibration curve and/or a tangibly embedded algorithm for applying said calibration curve to the coagulation time-related parameter or to a parameter derived therefrom. Thus, in an embodiment, the tangibly embedded algorithm implements the determination according to the method of the present invention. In an embodiment, the evaluation unit is adapted to determine a value of a coagulation-related parameter from said multitude of values of said transmission-related parameter.

Furthermore, the present disclosure relates to a kit for determining conjugated bilirubin and/or hemoglobin concentration comprising

i) thrombin; and
ii) at least one calibrator sample comprising conjugated bilirubin at a predetermined concentration or comprising hemoglobin at a predetermined concentration.

Moreover, the present disclosure relates to a kit for identifying a blood-derived sample having insufficient quality comprising

i) an agent eliciting coagulation; and
ii) at least one reference sample comprising a non-anticoagulant interferent in a predetermined, detectable amount.

Further, the present disclosure relates to a kit for determining a non-anticoagulant interferent, in an embodiment for determining conjugated bilirubin and/or hemoglobin and/or lipid, in a blood-derived sample of a subject comprising

i) an agent eliciting coagulation; and
ii) at least one reference sample comprising a non-anticoagulant interferent in a predetermined, detectable amount.

The term "kit", as used herein, refers to a collection of the aforementioned compounds, means or reagents of the present disclosure which may or may not be packaged together. The components of the kit may be comprised by separate housings (i.e. as a kit of separate parts) or, two or more components may be provided in a single housing. Moreover, it is to be understood that the kit may be used for practicing the methods referred to herein above. It is envisaged that components, e.g. all components, are provided in a ready-to-use manner for practicing the methods referred to above. In a disclosure, all or some of said components are provided in dried, such as in lyophilized form, wherein the component is reconstituted using a liquid such as an aqueous buffered solution. In a disclosure all or some of said components are provided in concentrated liquid form wherein the concentrated component is diluted using a liquid such as an aqueous buffered solution. In a disclosure all or some of said components are provided in ready-to-use form, i.e. in a state in which they can be used directly and, e.g., do not require dilution. In a disclosure, all or some of said components, in particular reference samples, are provided in frozen form, e.g. as frozen solutions. Further, the kit, may contain instructions for carrying out said methods and, if applicable, said reconstitution of dried reagents. The instructions can be provided by a user's manual in paper- or electronic form. In addition, the manual may comprise instructions for interpreting the results obtained when carrying out the aforementioned methods using the kit of the present invention.

In a disclosure, the kit further comprises bilirubin-oxidase. In a further disclosure, the kit further comprises at least one calibrator sample and/or at least one quality reference sample. In a further disclosure, the kit further comprises a compound selected from hexadimethrine bromide, protamine and heparinase; and/or comprises an anti-fibrinolytic agent, in a disclosure tranexamic acid or an anti-fibrinolytic derivative thereof or aprotinin or an anti-fibrinolytic derivative thereof. In a disclosure, the kit further comprises an agent providing calcium ions, in particular a solution comprising calcium ions at a concentration of at least 0.01 M, in an embodiment at least 0.02 M. In an embodiment, the kit further comprises thrombin.

In a disclosure, the kit further comprise at least one contact activator, in an embodiment silica, celite, kaolin, and/or ellagic acid, in particular silica gel or ellagic acid, in a disclosure further comprises phospholipids; in a disclosure, the kit further comprises (i) at least one reference sample comprising conjugated bilirubin at a predetermined concentration and/or (ii) at least one reference sample comprising hemoglobin at a predetermined concentration and/or (iii) at least one reference sample comprising lipids at a predetermined concentration. Thus, in a disclosure, the kit is a kit comprising APTT reagents, in an embodiment the APTT reagents as referred to herein, and at least one non-anticoagulant interferent calibrator sample.

In a further disclosure, the kit for determining conjugated bilirubin and/or hemoglobin concentration comprises a calibrator sample comprising conjugated bilirubin at a predetermined concentration of at least 1 mg/dL, in a disclosure at least 2.5 mg/dL, in a further disclosure at least 5 mg/dL. In a further disclosure, the kit for determining conjugated bilirubin and/or hemoglobin concentration comprises a calibrator sample comprising hemoglobin at a predetermined concentration of at least 20 mg/dL, in a disclosure at least 50 mg/dL, in a disclosure at least 100 mg/dL.

Also, the present disclosure relates to a use of the method for determining a non-anticoagulant interferent, of the device according to the present invention, and/or of the kit for quality control of blood-derived sample analysis.

In a disclosure, said quality control comprises excluding a sample being of insufficient quality from clinical chemistry analysis and/or from coagulation analysis. In a further disclosure a sample being of insufficient quality is a sample meeting at least one condition of being of insufficient quality as specified herein above.

Also, the present disclosure relates to a method for determining a bilirubin-corrected coagulation time-related parameter in a blood-derived sample of a subject, said method comprising a) contacting said sample with bilirubin oxidase (EC 1.3.3.5) and/or a beta-glucuronidase (EC 3.2.1.31), b) determining a value of a coagulation time-related parameter in the sample of step a); and, c) thereby, determining a bilirubin-corrected coagulation time-related parameter.

Moreover, the present disclosure relates to a method for determining conjugated bilirubin and a bilirubin-corrected coagulation time-related parameter in a subject, said method comprising a) determining a first value of a coagulation time-related parameter in a first blood-derived sample of said subject; b) contacting a second blood-derived sample of said subject with bilirubin oxidase (EC 1.3.3.5) and/or a beta-glucuronidase (EC 3.2.1.31), c) determining a second value of a coagulation time-related parameter in the sample of step b), d) comparing a parameter derived from the first value of the coagulation time-related parameter determined in a) to a reference; e) based on the result of comparison step d), determining conjugated bilirubin in said subject, f) determining a value of a coagulation time-related parameter in the second sample of step b); and, thereby g) determining conjugated bilirubin and a bilirubin-corrected coagulation time-related parameter in said subject. In a disclosure, the first and second blood-derived samples are aliquots of the same sample of the subject in such case.

The disclosure further discloses and proposes a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps a) to d) as indicated above may be performed by using a computer or a computer network, preferably by using a computer program.

The disclosure further relates to and proposes a computer program product having program code means, in order to perform the method according to the present invention in one or more of the embodiments enclosed herein when the program is executed on a computer or computer network. Specifically, the program code means may be stored on a computer-readable data carrier.

Further, the disclosure relates to and proposes a data carrier having a data structure stored thereon, which, after loading into a computer or computer network, such as into a working memory or main memory of the computer or computer network, may execute the method according to one or more of the embodiments disclosed herein.

The disclosure further proposes and discloses a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the disclosure proposes and discloses a modulated data signal which contains instructions readable by a computer system or computer network, for performing the method according to one or more of the embodiments disclosed herein.

Preferably, referring to the computer-implemented aspects, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present disclosure further discloses:

- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and

- a computer program product having program code means, wherein the program code means can be stored or are stored on a storage medium, for performing the method according to one of the embodiments described in this description, if the program code means are executed on a computer or on a computer network.

Figure Legends

Fig. 1: Calibration curve for the determination of conjugated bilirubin (bilic) using an APTT reagent on a Roche t711 coagulation analyzer. A normal plasma pool was spiked with increasing amounts of conjugated bilirubin.

Fig. 2: Calibration curve for the determination of conjugated bilirubin (bilic) using an APTT reagent on a Roche t711 coagulation analyzer. A pathological plasma pool was spiked with increasing amounts of conjugated bilirubin.

Fig. 3: Calibration curve for the determination of unconjugated bilirubin (biliu) using an APTT reagent on a Roche t711 coagulation analyzer. A normal plasma pool was spiked with increasing amounts of unconjugated bilirubin.

Fig. 4: Calibration curve for the determination of hemoglobin using an APTT reagent on a Roche t711 coagulation analyzer. A normal plasma was spiked with increasing amounts of hemoglobin and subjected to an activated partial thromboplastin time assay using Roche APTT LS reagent.

Fig. 5: Calibration curve for the determination of triglycerides using an APTT reagent on a Roche t711 coagulation analyzer. A normal plasma was spiked with increasing amounts of Intralipid® and subjected to an activated partial thromboplastin time assay using Roche APTT LS reagent.

Fig. 6: Calibration curve for the determination of triglycerides using an APTT reagent on a Roche t711 coagulation analyzer. A pathological plasma pool was spiked with increasing amounts of Intralipid® and subjected to an activated partial thromboplastin time assay using Roche APTT LS reagent.

Fig. 7: Calibration curve for the determination of conjugated bilirubin (bilic) using a thrombin time reagent on a Roche Cobas t711 coagulation analyzer. A normal plasma pool was spiked with increasing amounts of conjugated bilirubin.

Fig. 8: Calibration curve for the determination of conjugated bilirubin (bilic) using a thrombin time reagent on a Roche Cobas t711 coagulation analyzer. A pathological plasma pool was spiked with increasing amounts of conjugated bilirubin

Fig. 9: Calibration curve for the determination of unconjugated bilirubin (biliu) using a thrombin time reagent on a Roche Cobas t711 coagulation analyzer. A normal plasma pool was spiked with increasing amounts of unconjugated bilirubin.

Fig. 10: Calibration curve for the determination of hemoglobin using a thrombin time reagent on a Roche Cobas t711 coagulation analyzer. A pathological plasma was spiked with increasing amounts of hemoglobin

The following Examples shall merely illustrate the invention. They shall not be construed, whatsoever, to limit the scope of the invention. Examples 1 to 3 and 5 are comparative Examples.

**Example 1:** Quantification of conjugated bilirubin (bilic) in a sample (APTT assay)

Unexpectedly it was found that conjugated bilirubin (bilic) can be quantified on an automated coagulation analyzer (Roche cobas t711) using a commercially available activated partial thromboplastin time reagent (Roche APTT LS Reagent, in development). Addition of increasing concentrations of conjugated bilirubin to a normal plasma sample showed a linear positive correlation between the amount of conjugated bilirubin added and the resulting coagulation time in [s]. This means the clotting time of APTT assay was systematically prolonged dependent on the amount of spiked conjugated bilirubin (Table 1).

Table 1: Quantification of conjugated bilirubin (bilic) using an APTT reagent. Conjugated bilirubin was spiked to a normal plasma in increasing amounts. Subsequently this plasma was subjected to an activated partial thromboplastin time assay using an APTT reagent. Assay: Roche APTT LS on Roche Cobas t711 coagulation analyzer, sample: Normal plasma pool.

| Conj. Bilirubin [mg/dL] | APTT result [s] |
|---|---|
| 0.00 | 27.60 |
| 2.00 | 27.90 |
| 4.00 | 28.40 |
| 6.00 | 29.00 |
| 8.00 | 29.70 |
| 9.00 | 30.00 |
| 10.00 | 30.50 |
| 11.00 | 30.70 |
| 12.00 | 31.10 |

(continued)

| Conj. Bilirubin [mg/dL] | APTT result [s] |
|---|---|
| 13.00 | 31.50 |
| 15.00 | 32.20 |

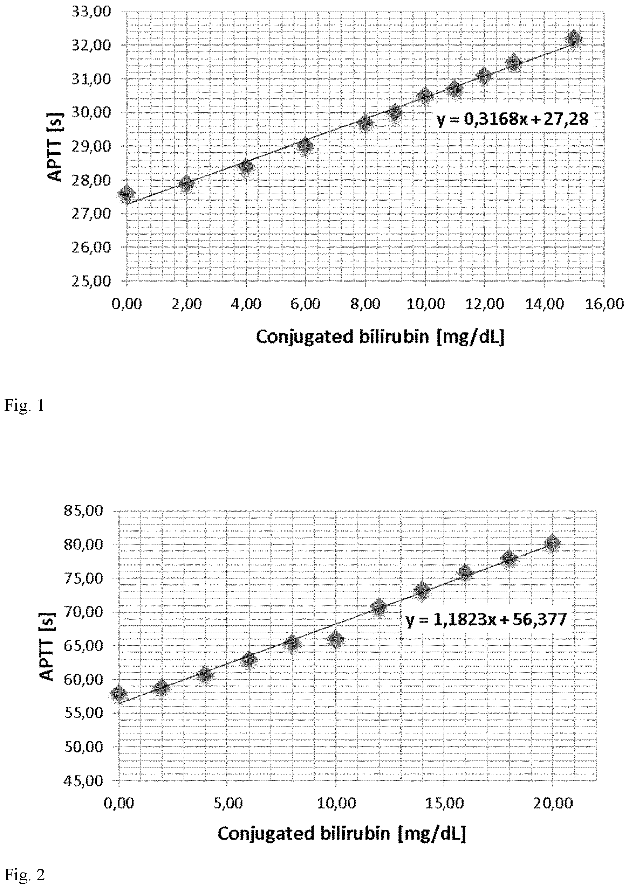

This shows that the APTT assay in combination with a normal plasma sample can be used to determine the unknown amount of conjugated bilirubin in a plasma sample. The graphic representation of the data shown in Table 1 is a calibration curve for a bilirubin determination assay (Figure 1). Similar results were obtained when a pathological plasma pool spiked with increasing concentrations of conjugated bilirubin was used (Figure 2).

Using the calibration curve and the respective equation from Figure 1, an unknown amount of conjugated bilirubin ($\geq$ 2 mg/dL) can be derived from an APTT result in seconds. Example:

$$\frac{y - 27.28}{0.3168} = x$$

An APTT result of 31 s would result in a conjugated bilirubin concentration x = 11.74 mg/dL.

The calibration curve shown above was generated by spiking increasing amount of bilic to a normal plasma sample. Similar results were obtained when a pathological plasma sample was spiked with increasing amounts of bilic and subjected to an APTT assay. This demonstrates that there is a systematic in the dose-dependency between coagulation times and bilirubin concentrations.

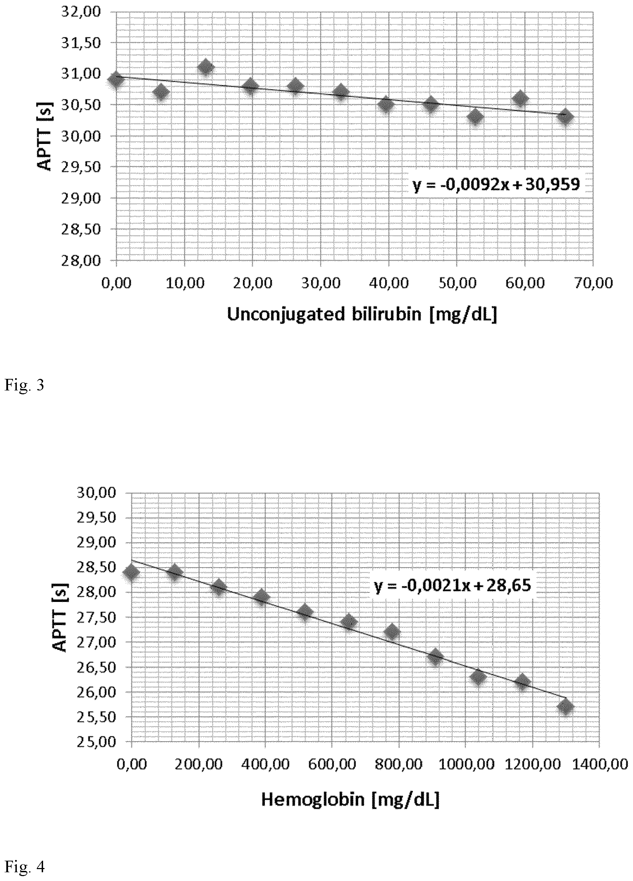

In contrast to the results obtained with conjugated bilirubin (bilic) it could be shown that unconjugated bilirubin (biliu) influences APTT results only marginally, even in high concentrations (Table 2 and Figure 3).

Table 2: Quantification of unconjugated bilirubin (biliu) using an APTT reagent. Unconjugated bilirubin was spiked to a normal plasma in increasing amounts. Subsequently this plasma was subjected to an APTT assay using Roche APTT LS reagent. APTT LS on Roche Cobas t711 coagulation analyzer ; sample: Normal plasma pool.

| Unconj. Bilirubin [mg/dL] | APTT result [s] |
|---|---|
| 0.00 | 30.90 |
| 6.60 | 30.70 |
| 13.20 | 31.10 |
| 19.80 | 30.80 |
| 26.40 | 30.80 |
| 33.00 | 30.70 |
| 39.60 | 30.50 |
| 46.20 | 30.50 |
| 52.80 | 30.30 |
| 59.40 | 30.60 |

(continued)

| Unconj. Bilirubin [mg/dL] | APTT result [s] |
|---|---|
| 66.00 | 30.30 |

This shows that an APTT reagent based bilirubin assay can be used to determine conjugated bilirubin without substantial interference by unconjugated bilirubin. This assay allows the discrimination between conjugated and unconjugated bilirubin, which is not trivial when conventional methods are used.

**Example 2:** Quantification of hemoglobin in a sample (APTT assay)

Essentially analogous to the methods and the examples described above, it was found that the same APTT reagent (Roche APTT LS) can be used to quantify hemoglobin concentration in a citrate plasma sample on an automated coagulation analyzer (Roche Cobas t711 coagulation analyzer, currently in development). Addition of increasing concentration of hemoglobin to a pathological plasma sample showed a linear negative correlation between the amount of hemoglobin added and the resulting coagulation time in [s]. In contrast to the bilirubin quantification method which showed a dosed-dependent prolongation of clotting time with increasing bilic concentration, the clotting time of the APTT assay was systematically shortened dependent on the amount of hemoglobin spiked (Table 3).

Table 3: Quantification of hemoglobin using an APTT reagent. Hemoglobin was spiked to a normal citrated plasma in increasing amounts. Subsequently this plasma was subjected to an activated partial thromboplastin time assay using an APTT reagent. Assay: Roche APTT LS on Roche Cobas t711 coagulation analyzer, sample: normal plasma pool.

| Hemoglobin [mg/dL] | APTT result [s] |
|---|---|
| 0.00 | 28.40 |
| 130.00 | 28.40 |
| 260.00 | 28.10 |
| 390.00 | 27.90 |
| 520.00 | 27.60 |
| 650.00 | 27.40 |
| 780.00 | 27.20 |
| 910.00 | 26.70 |
| 1040.00 | 26.30 |
| 1170.00 | 26.20 |
| 1300.00 | 25.70 |

This shows that the APTT assay in combination with a normal plasma sample is suited to determine the unknown amount of hemoglobin in a plasma sample. The graphic representation of the data shown in Table 3 is a calibration curve for a hemoglobin determination assay (Figure 4).

Using the calibration curve and the respective equation from Figure 4, an unknown amount of hemoglobin could be derived from an APTT result in seconds. Example:

$$\frac{y - 28.65}{-0.0021} = x$$

An APTT result of 27 s would result in a hemoglobin concentration x = 785.71 mg/dL. Similar to the bilirubin quantification method described above, the APTT reagent used can be modified in a way that heparin neutralizers are added and thereby the specificity of the APTT result for the quantification of hemoglobin is systematically improved.

**Example 3: Quantification of triglycerides in a sample (APTT assay)**

Essentially analogous to the methods and the examples described above, it was found that the same APTT reagent (Roche APTT LS) can be used to quantify triglyceride concentrations in a citrate plasma sample on an automated coagulation analyzer (Roche Cobas t711). Addition of increasing concentrations of Intralipid® (emulsion of soy bean oil (20 % (w/v), emulgated by purified egg phospholipids and glycerin) to a normal or to a pathological plasma sample showed a linear negative correlation between the amount of Intralipid® added and the resulting coagulation time in [s]. In contrast to the bilirubin quantification method which showed a dosed-dependent prolongation of clotting time with increasing bilic concentration, the clotting time of the APTT assay was systematically shortened dependent on the amount of Intralipid® spiked (Table 4).

Table 4: Quantification of triglycerides using an APTT reagent. Intralipid® was spiked to a normal citrated plasma in increasing amounts. Subsequently this plasma was subjected to an activated partial thromboplastin time assay using an APTT reagent. Assay: Roche APTT LS on t711 coagulation analyzer, sample: normal plasma pool.

| Intralipid® [mg/dL] | APTT result [s] |
|---|---|
| 0.00 | 28.40 |
| 80.00 | 28.30 |
| 160.00 | 28.10 |
| 240.00 | 28.00 |
| 320.00 | 27.80 |
| 400.00 | 27.60 |
| 480.00 | 27.60 |

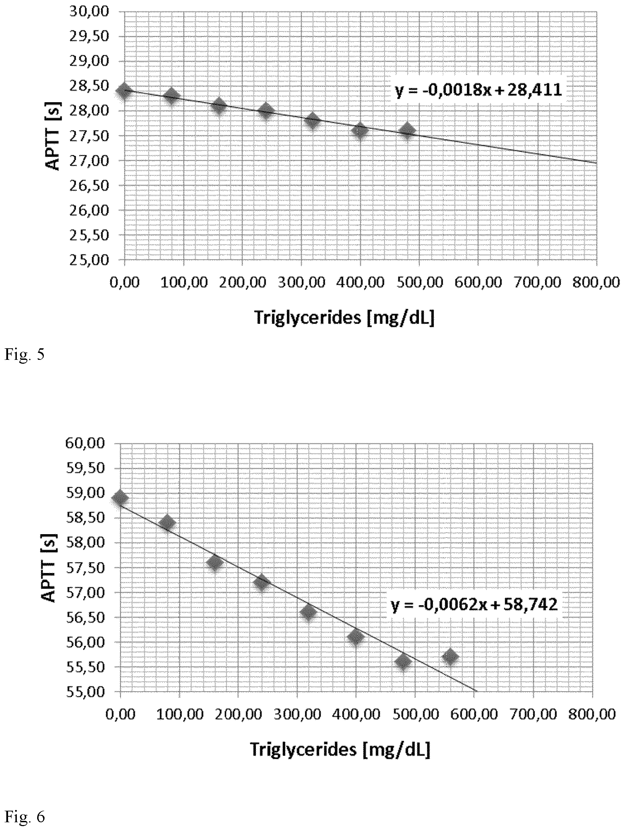

This shows that the APTT assay in combination with a normal plasma sample is suited to determine the unknown amount of triglycerides in a plasma sample. The graphic representation of the data shown in Table 4 is a calibration curve for a hemoglobin determination assay (Figure 5).

Using the calibration curve and the respective equation from Figure 5, an unknown amount of triglyceride can be derived from an APTT result in seconds. Example:

$$\frac{y - 28.411}{-0.0018} = x$$

An APTT result of 27.9 s would result in a hemoglobin concentration x = 283.88 mg/dL. Similar results were obtained when a pathological plasma sample was spiked with increasing amounts of Intralipid® and subjected to an APTT assay. This demonstrates that there is a systematic in the dose-dependency between coagulation times and Intralipid® concentrations (Figure 6).

Similar to the bilirubin quantification method described above, the APTT reagent used can be modified in a way that heparin neutralizers are added and thereby the specificity of the APTT result for the quantification of hemoglobin is systematically improved.

**Example 4: Quantification of conjugated bilirubin (bilic) in a sample (Thrombin Time assay)**

Unexpectedly it was found that conjugated bilirubin (bilic) can be quantified on an automated coagulation analyzer (Roche Cobas t711 coagulation analyzer, currently in development) using a thrombin reagent (Roche Thrombin Time Reagent, in development)). Addition of increasing concentrations of conjugated bilirubin to a normal plasma sample showed a linear positive correlation between the amount of conjugated bilirubin added and the resulting coagulation time in [s]. This means the clotting time of thrombin time (TT) assay was systematically prolonged dependent on the amount of spiked conjugated bilirubin (Table 5).

Table 5: Quantification of conjugated bilirubin (bilic) using a thrombin reagent. Conjugated bilirubin was spiked to a normal plasma in increasing amounts. Subsequently this plasma was subjected to a thrombin time assay using a commercial thrombin reagent. Assay: Thrombin time on Roche Cobas t711 coagulation analyzer (currently in development), sample: Normal plasma pool

| Conj. Bilirubin [mg/dL] | TT result [s] |
|---|---|
| 0.00 | 16.70 |
| 1.00 | 16.70 |
| 2.00 | 17.40 |
| 3.00 | 17.80 |
| 4.00 | 18.50 |
| 5.00 | 19.20 |

(continued)

| Conj. Bilirubin [mg/dL] | TT result [s] |
|---|---|
| 6.00 | 19.50 |
| 7.00 | 20.00 |
| 8.00 | 20.50 |
| 9.00 | 21.30 |
| 10.00 | 21.70 |

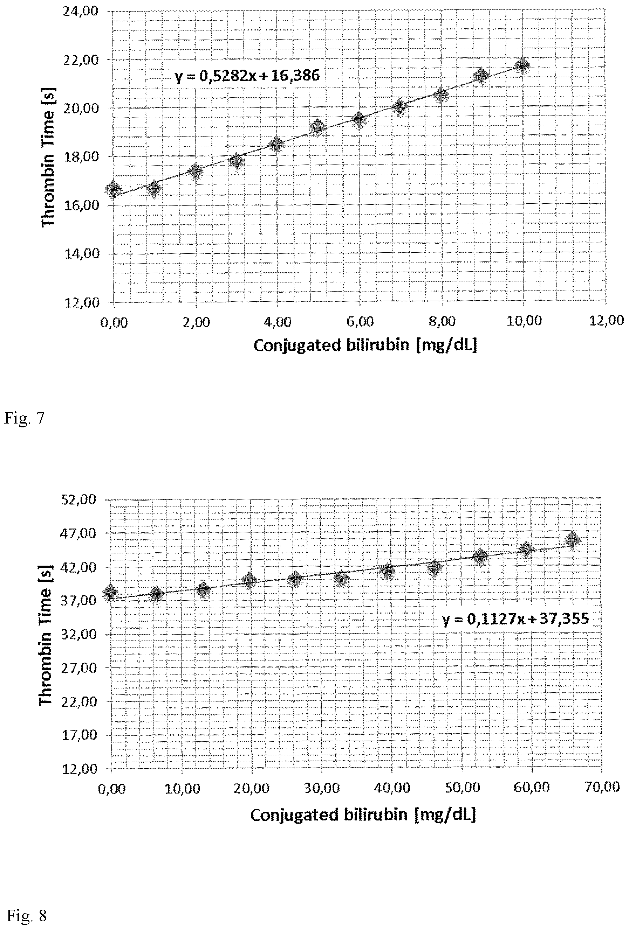

This shows that the thrombin time assay (the active ingredient in a TT assay is the enzyme thrombin) in combination with a normal plasma sample can be used to determine the unknown amount of conjugated bilirubin in a plasma sample. The graphic representation of the data shown in Table 5 is a calibration curve for a bilirubin determination assay (Figure 7).

Using the calibration curve and the respective equation from Figure 7, an unknown amount of conjugated bilirubin ($\geq$ 1 mg/dL) could be derived from a TT result in seconds. Example:

$$\frac{y - 16.386}{0.5282} = x$$

A TT result of 19 s would result in a conjugated bilirubin concentration x = 4.95 mg/dL.

The calibration curve shown above was generated by spiking increasing amount of bilic to a normal plasma sample. Similar results were obtained when a pathological plasma sample was spiked with increasing amounts of bilic and subjected to a thrombin time assay (Figure 8). This demonstrates that there is a systematic in the dose-dependency between coagulation times and bilirubin concentrations.

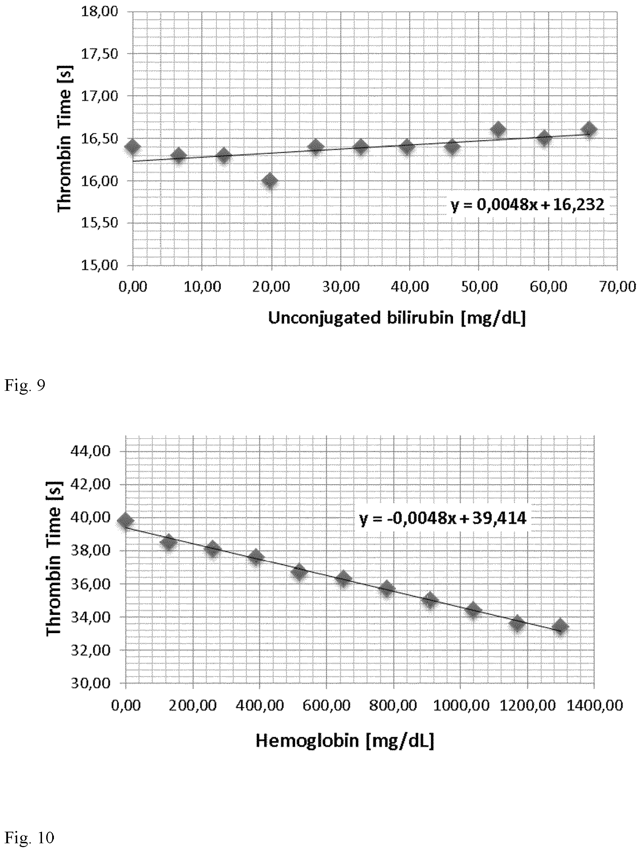

In contrast to the results obtained with conjugated bilirubin (bilic) it could be shown that unconjugated bilirubin (biliu) does not seem to influence thrombin time results, even in high concentrations (Table 6 and Figure 9).

Table 6: Quantification of unconjugated bilirubin (biliu) using a thrombin reagent. Unconjugated bilirubin was spiked to a normal plasma in increasing amounts. Subsequently this plasma was subjected to a thrombin time assay using a commercial thrombin reagent. Assay: Thrombin time on Roche Cobas t711 coagulation analyzer, sample: Normal plasma pool.

| Unconj. Bilirubin [mg/dL] | TT result [s] |
|---|---|
| 0.00 | 16.40 |
| 6.60 | 16.30 |
| 13.20 | 16.30 |
| 19.80 | 16.00 |
| 26.40 | 16.40 |
| 33.00 | 16.40 |
| 39.60 | 16.40 |

(continued)

| Unconj. Bilirubin [mg/dL] | TT result [s] |
|---|---|
| 46.20 | 16.40 |
| 52.80 | 16.60 |
| 59.40 | 16.50 |
| 66.00 | 16.60 |

This shows that a thrombin reagent based bilirubin assay can be used to determine conjugated bilirubin without substantial interference by unconjugated bilirubin. This assay allows the discrimination between conjugated and unconjugated bilirubin, which is not trivial when conventional methods are used.

**Example 5:** Quantification of hemoglobin in a sample (TT assay)

Essentially analogous to the methods of Example 4 described above, it was found that the same thrombin reagent (Roche Thrombin Time Reagent) can be used to quantify hemoglobin concentration in a citrated plasma sample on an automated coagulation analyzer (Roche Cobas t711 coagulation analyzer, currently in development). Addition of increasing concentration of hemoglobin to a pathological plasma sample showed a linear negative correlation between the amount of hemoglobin added and the resulting coagulation time in [s]. In contrast to the bilirubin quantification method which showed a dosed-dependent prolongation of clotting time with increasing bilic concentration, the clotting time of the thrombin time assay was systematically shortened dependent on the amount of hemoglobin spiked (Table 7).

Table 7: Quantification of hemoglobin using a thrombin reagent. Hemoglobin was spiked to a normal citrated plasma in increasing amounts. Subsequently this plasma was subjected to a thrombin time (TT) assay using a commercial thrombin reagent. Assay: Thrombin time on Roche Cobas t711 coagulation analyzer, sample: pathological plasma pool.

| Hemoglobin [mg/dL] | TT result [s] |
|---|---|
| 0.00 | 39.80 |
| 130.00 | 38.50 |
| 260.00 | 38.10 |
| 390.00 | 37.60 |
| 520.00 | 36.70 |
| 650.00 | 36.30 |
| 780.00 | 35.70 |
| 910.00 | 35.00 |
| 1040.00 | 34.40 |
| 1170.00 | 33.60 |
| 1300.00 | 33.40 |

This shows that the thrombin time assay in combination with a pathological plasma sample is suited to determine the unknown amount of hemoglobin in a plasma sample. The graphic representation of the data shown in Table 7 is a calibration curve for a hemoglobin determination assay (Figure 10).

Using the calibration curve and the respective equation from Figure 10, an unknown amount of hemoglobin could be derived from a TT result in seconds. Example:

$$\frac{y - 39.414}{-0.0048} = x$$

A TT result of 35 s would result in a hemoglobin concentration x = 919.58 mg/dL.

Similar to the bilirubin quantification method described above, the thrombin reagent used can be modified in a way, that heparin neutralizers would be added and thereby the specificity of the thrombin time result for the quantification of hemoglobin are systematically improved.

**Claims**

1. A method for determining conjugated bilirubin in a blood-derived sample of a subject, said method comprising

   a) determining a value of thrombin time in said sample;
   b) comparing the value of thrombin time determined in a) to a value of thrombin time determined in at least one reference sample; and
   c) based on the result of comparison step b), determining conjugated bilirubin in a blood-derived sample of a subject,

   wherein step a) is determining a value of thrombin time in said sample with a photo-optical assay.

2. The method of claim 1, wherein step a) is determining a value of thrombin time in said sample by turbidimetry.

3. The method of claim 1 or 2, wherein step a) comprises the substeps

   a1) contacting said sample with thrombin;
   a2) determining a first value of a transmission-related parameter in said sample at a first time point after contacting the sample with thrombin;
   a3) determining a second value of a transmission-related parameter in said sample at a second time point after contacting the sample with thrombin; and
   a4) determining a value of thrombin time based on the results of substeps a2) and a3).

4. The method of any one of claims 1 to 3, wherein step b) comprises substeps b

   1) providing at least two, in an embodiment at least three, in a further embodiment at least five, calibrator samples having predetermined, non-identical concentrations of conjugated bilirubin; b2) determining a value of thrombin time for each of said calibrator samples, in an embodiment according to the substeps of step a); and b3) providing a calibration curve based on the values determined in substep b2).

5. The method of any one of claims 1 to 4, wherein said sample is a citrate plasma sample.

6. The method of any one of claims 1 to 5, wherein said method further comprises contacting an aliquot of said sample with bilirubin oxidase EC 1.3.3.5 and/or a beta-glucuronidase EC 3.2.1.31 and determining a value of thrombin time in said treated aliquot, in an embodiment, wherein said method further comprises determining a value of total bilirubin and calculating free bilirubin from the values determined for total bilirubin and conjugated bilirubin.

7. The method of any one of claims 1 to 6, wherein said subject is a subject with an increased risk for developing bilirubin encephalopathy and/or wherein said subject is a subject not being administered anticoagulation therapy.

8. The method of any one of claims 1 to 7, wherein said determination is quantitative determination.

9. A method for identifying a blood-derived sample having insufficient quality, said method comprising

   A) determining conjugated bilirubin according to any one of claims 1 to 8;
   B) comparing the result of step A) to a quality reference; and
   C) based on the result of comparison step B), identifying a sample having insufficient quality.

10. The method of claim 9, wherein said method comprises evaluating at least one of the following conditions and wherein said sample is identified as having insufficient quality if at least one of the following conditions is met:

   i) the value of a coagulation time-related parameter in said sample is lower than a quality reference;
   ii) the value of the coagulation time-related parameter in said sample is higher than a quality reference and the value of the coagulation time-related parameter is significantly lower after treatment of said sample with bilirubin oxidase and/or a beta-glucuronidase; and
iii) the concentration of conjugated bilirubin determined is higher than 1 mg/dl, in an embodiment higher than 2.5 mg/dL, in an embodiment, wherein a sample identified as having insufficient quality is excluded from clinical chemistry analysis.

11. A device for determining conjugated bilirubin in a blood-derived sample, comprising an analysis unit and an evaluation unit, wherein

(I) said analysis unit is adapted for determining a value of thrombin time in said sample, wherein said value of thrombin time is determined by a photo-optical method, and
(II) said evaluation unit comprises a memory unit comprising tangibly embedded an algorithm adapted for performing steps b) and c) of the method of claim 1.

**Patentansprüche**

1. Verfahren zur Bestimmung von konjugiertem Bilirubin in einer aus Blut stammenden Probe eines Patienten, wobei das Verfahren Folgendes umfasst:

a) Bestimmen eines Werts der Thrombinzeit in der Probe;
b) Vergleichen des in a) bestimmten Werts der Thrombinzeit mit einem in mindestens einer Referenzprobe bestimmten Wert der Thrombinzeit; und
c) Bestimmen von konjugiertem Bilirubin in einer aus Blut stammenden Probe eines Patienten auf Basis des Ergebnisses des Vergleichsschritts b),

wobei es sich bei Schritt a) um das Bestimmen eines Werts der Thrombinzeit in der Probe mit einer photooptischen Methode handelt.

2. Verfahren nach Anspruch 1, wobei es sich bei Schritt a) um das Bestimmen eines Werts der Thrombinzeit durch Turbidimetrie handelt.

3. Verfahren nach Anspruch 1 oder 2, wobei Schritt a) die folgenden Unterschritte umfasst:

a1) Inkontaktbringen der Probe mit Thrombin;
a2) Bestimmen eines ersten Werts eines transmissionsbezogenen Parameters in der Probe zu einem ersten Zeitpunkt nach dem Inkontaktbringen der Probe mit Thrombin;
a3) Bestimmen eines zweiten Werts eines transmissionsbezogenen Parameters in der Probe zu einem zweiten Zeitpunkt nach dem Inkontaktbringen der Probe mit Thrombin; und
a4) Bestimmen eines Werts der Thrombinzeit auf Basis der Ergebnisse der Unterschritte a2) und a3).

4. Verfahren nach einem der Ansprüche 1 bis 3, wobei Schritt b) die folgenden Unterschritte umfasst:

b1) Bereitstellen von mindestens zwei, in einer Ausführungsform mindestens drei, in einer weiteren Ausführungsform mindestens fünf, Kalibrierproben mit vorgegebenen, nicht identischen Konzentrationen konjugierten Bilirubins;
b2) Bestimmen eines Werts der Thrombinzeit für jede der Kalibrierproben, in einer Ausführungsform gemäß der Unterschritte von Schritt a); und
b3) Bereitstellen einer Kalibrierkurve auf Basis der in Unterschritt b) bestimmten Werte.

5. Verfahren nach einem der Ansprüche 1 bis 4, wobei es sich bei der Probe um eine Citratplasmaprobe handelt.

6. Verfahren nach einem der Ansprüche 1 bis 5, wobei das Verfahren ferner das Inkontaktbringen eines Aliquots der Probe mit Bilirubinoxidase EC 1.3.3.5 und/oder Beta-Glucuronidase EC 3.2.1.31 und das Bestimmen eines Werts der Thrombinzeit in dem behandelten Aliquot umfasst, wobei das Verfahren in einer Ausführungsform ferner das Bestimmen eines Werts des Gesamtbilirubins und das Berechnen des freien Bilirubins aus den für das Gesamtbilirubin und das konjugierte Bilirubin bestimmten Werten umfasst.

7. Verfahren nach einem der Ansprüche 1 bis 6, wobei es sich bei dem Patienten um einen Patienten mit erhöhtem Risiko zur Ausbildung einer Bilirubin-Encephalopathie handelt und/oder wobei es sich bei dem Patienten um einen Patienten handelt, dem keine Antikoagulationstherapie verabreicht wird.

8. Verfahren nach einem der Ansprüche 1 bis 7, wobei es sich bei der Bestimmung um eine quantitative Bestimmung handelt.

9. Verfahren zum Ermitteln einer aus Blut stammenden Probe mit unzureichender Qualität, wobei das Verfahren Folgendes umfasst:

A) Bestimmen des konjugierten Bilirubins nach einem der Ansprüche 1 bis 8;
B) Vergleichen des Ergebnisses aus Schritt A) mit einer Qualitätsreferenz; und
C) Ermitteln einer Probe mit unzureichender Qualität auf Basis des Ergebnisses des Vergleichsschritts B).

**10.** Verfahren nach Anspruch 9, wobei das Verfahren das Beurteilen von mindestens einer der folgenden Vorgaben umfasst und wobei die Probe als eine unzureichende Qualität aufweisend erkannt wird, wenn mindestens eine der folgenden Vorgaben erfüllt ist:

i) der Wert eines Koagulationszeit-bezogenen Parameters in der Probe ist geringer als eine Qualitätsreferenz;

ii) der Wert des Koagulationszeit-bezogenen Parameters in der Probe ist höher als eine Qualitätsreferenz und der Wert des Koagulationszeit bezogenen Parameters ist nach der Behandlung der Probe mit Bilirubinoxidase und/oder Beta-Glucuronidase deutlich geringer; und

iii) die bestimmte Konzentration konjugierten Bilirubins ist höher als 1 mg/dl, in einer Ausführungsform höher als 2,5 mg/dl, wobei eine als Probe mit unzureichender Qualität ermittelte Probe in einer Ausführungsform von der klinisch-chemischen Analyse ausgeschlossen wird.

**11.** Vorrichtung zum Bestimmen von konjugiertem Bilirubin in einer aus Blut stammenden Probe, die eine Analyseeinheit und eine Auswerteeinheit umfasst, wobei

(I) die Analyseeinheit zum Bestimmen eines Werts der Thrombinzeit in der Probe eingerichtet ist, wobei der Wert der Thrombinzeit durch ein photooptisches Verfahren bestimmt wird, und

(II) die Auswerteeinheit eine Speichereinheit umfasst, die einen greifbar eingebetteten Algorithmus, der zur Durchführung der Schritte b) und c) des Verfahrens nach Anspruch 1 eingerichtet ist, umfasst.

## Revendications

**1.** Procédé destiné à déterminer la bilirubine conjuguée dans un échantillon dérivé du sang d'un sujet, ledit procédé comprenant les étapes consistant à

a) déterminer une valeur du temps de thrombine dans ledit échantillon ;
b) comparer la valeur du temps de thrombine déterminée dans l'étape a) avec une valeur du temps de thrombine déterminée dans au moins un échantillon de référence ; et
c) sur la base du résultat de la comparaison de l'étape b), déterminer la bilirubine conjuguée dans un échantillon dérivé du sang d'un sujet,

dans lequel l'étape a) détermine une valeur du temps de thrombine dans ledit échantillon avec un dosage photo-optique.

**2.** Procédé de la revendication 1, dans lequel l'étape a) détermine une valeur du temps de thrombine dans ledit échantillon par turbidimétrie.

**3.** Procédé des revendications 1 ou 2, dans lequel l'étape a) comprend les sous-étapes consistant à

a1) mettre en contact ledit échantillon avec de la thrombine ;
a2) déterminer une première valeur d'un paramètre lié à la transmission dans ledit échantillon, à un premier point dans le temps, après avoir mis en contact l'échantillon avec la thrombine ;
a3) déterminer une deuxième valeur d'un paramètre lié à la transmission dans ledit échantillon, à un deuxième point dans le temps, après avoir mis en contact l'échantillon avec la thrombine ; et
a4) déterminer une valeur du temps de thrombine sur la base des résultats des sous-étapes a2) et a3).

**4.** Procédé de l'une quelconque des revendications 1 à 3, dans lequel l'étape b) comprend les sous-étapes consistant à

b1) fournir au moins deux, dans un mode de réalisation au moins trois, dans un mode de réalisation en outre au moins cinq, échantillons étalons ayant des concentrations en bilirubine conjuguée prédéterminées, non identiques ;
b2) déterminer une valeur du temps de thrombine pour chacun desdits échantillons étalons, dans un mode de réalisation selon les sous-étapes de l'étape a) ; et
b3) fournir une courbe d'étalonnage sur la base des valeurs déterminées dans la sous-étape b2).

**5.** Procédé de l'une quelconque des revendications 1 à 4, dans lequel ledit échantillon est un échantillon de plasma citraté.

**6.** Procédé de l'une quelconque des revendications 1 à 5, ledit procédé comprenant en outre les étapes de mise en contact d'une aliquote dudit échantillon avec une bilirubine oxydase EC 1.3.3.5 et/ou une bêta-glucuronidase EC 3.2.1.31 et de détermination d'une valeur du temps de thrombine dans ladite aliquote traitée, dans un mode de réalisation, ledit procédé comprenant en outre les étapes de détermination d'une valeur de la bilirubine totale et de calcul de la bilirubine libre à partir des valeurs déterminées pour la bilirubine totale et la bilirubine conjuguée.

**7.** Procédé de l'une quelconque des revendications 1 à 6, dans lequel ledit sujet est un sujet présentant un risque accru de développer une encéphalopathie bilirubinique et/ou dans lequel ledit sujet est un sujet à qui on n'a pas administré de thérapie anticoagulante.

**8.** Procédé de l'une quelconque des revendications 1 à 7, dans lequel ladite détermination est une détermination quantitative.

**9.** Procédé destiné à identifier un échantillon dérivé de sang ayant une qualité insuffisante, ledit procédé comprenant les étapes consistant à

A) déterminer la bilirubine conjuguée selon l'une quelconque des revendications 1 à 8 ;
B) comparer le résultat de l'étape A) avec une référence de qualité ; et
C) sur la base du résultat de l'étape B) de comparaison, identifier un échantillon ayant une qualité insuffisante.

**10.** Procédé de la revendication 9, ledit procédé comprenant une évaluation d'au moins l'une des conditions suivantes et dans lequel ledit échantillon est identifié comme ayant une qualité insuffisante si au moins l'une des conditions suivantes est satisfaite :

i) la valeur d'un paramètre lié au temps de coagulation dans ledit échantillon est inférieure à une référence de qualité ;
ii) la valeur du paramètre lié au temps de coagulation dans ledit échantillon est supérieure à une référence de qualité et la valeur du paramètre lié au temps de coagulation est significativement inférieure après un traitement dudit échantillon avec une bilirubine oxydase et/ou une bêta-glucuronidase ; et
iii) la concentration en bilirubine conjuguée déterminée est supérieure à 1 mg/dl, dans un mode de réalisation supérieure à 2,5 mg/dl, dans un mode de réalisation, dans lequel un échantillon identifié comme ayant une qualité insuffisante est exclu de l'analyse chimique clinique.

**11.** Dispositif destiné à déterminer la bilirubine conjuguée dans un échantillon dérivé de sang, comprenant une unité d'analyse et une unité d'évaluation, dans lequel

(I) ladite unité d'analyse est adaptée pour déterminer une valeur du temps de thrombine dans ledit échantillon, dans lequel ladite valeur du temps de thrombine est déterminée par un procédé photo-optique, et
(II) ladite unité d'évaluation comprend une unité de mémoire comprenant un algorithme, incorporé de manière tangible, adapté pour exécuter les étapes b) et c) du procédé de la revendication 1.

Fig. 1

Fig. 2

Fig. 3

Fig. 4

Fig. 5

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6326208 B1

**Non-patent literature cited in the description**

- **KOPEC et al.** *J Clin Path,* 1961, vol. 14, 478
- **SHIN et al.** *Ann Lab Med,* 2014, vol. 34, 307
- **BOYD et al.** *Clin Chim Acta,* 2015, vol. 450, 3
- **WALTERS ; GERARDE.** *Microchemical Journal,* 1970, vol. 15, 231
- **VALDES et al.** *Annal. Clin. Lab. Science,* 1979, vol. 9 (3), 251
- **IWATANI et al.** *Scientific Reports,* 2016
- **HUBBUCH et al.** *Clin. Lab.,* 1996, vol. 42, 637-640